# EUROPEAN PATENT SPECIFICATION

(11) **EP 0 652 775 B1**
(45) Date of publication and mention of the grant of the patent: **19.04.2000**
(21) Application number: 93917326.6
(22) Date of filing: 23.07.1993
(51) Int. Cl.: A61K 47/48

(54) **TARGETING OF LIPOSOMES TO THE BLOOD-BRAIN BARRIER**
ZIELGERICHTE LIPOSOME ZUR BLUT-HIRNE SCHRANKE
CIBLAGE DE LIPOSOMES SUR LA BARRIERE HEMATO-ENCEPHALIQUE

(30) Priority: 27.07.1992 US 921199
(43) Date of publication of application: 17.05.1995
(73) Proprietor: THE GOVERNMENT OF THE UNITED STATES OF AMERICA as represented by the SECRETARY OF THE DEPARTMENT OF HEALTH AND HUMAN SERVICES, Bethesda, MD 20892-9902 (US)
(72) Inventor: MICKLUS, Michael J., Philadelphia, PA 19104 (US); GREIG, Nigel H., Silver Springs, MD 20906 (US); RAPOPORT, Stanley I., Washington, DC 20016 (US)
(74) Representative: Thomson, Paul Anthony
(86) International application number: US9306942
(87) International publication number: WO9402178

(56) References cited:
- EP-A- 0 520 499
- WO-A-91/03259
- WO-A-91/04014
- WO-A-91/19501
- WO-A-93/10819
- PROCEEDINGS OF THE NATIONAL ACADEMY OF SCIENCES OF USA vol. 88 , June 1991 , WASHINGTON US pages 4771 - 4775 P. M. FRIDEN ET AL. 'ANTI-TRANSFERRIN RECEPTOR ANTIBODY AND ANTIBODY-DRUG CONJUGATES CROSS THE BLOOD-BRAIN BARRIER.' cited in the application
- CHEMICAL ABSTRACTS, vol. 112, no. 2, 8 January 1990, Columbus, Ohio, US; abstract no. 11839n, & BLOOD vol. 74, no. 6 , 1989 pages 2043 - 2052 K. M. HEGE 'COMPARISON OF ANTI-TAC AND ANTI-TRANSFERRIN RECEPTOR-CONJUGATED LIPOSOMES FOR SPECIFIC DRUG DELIVERY TO ADULT T-CELL LEUKEMIA.'
- BIOLOGICAL ABSTRACTS vol. 89 1989, Philadelphia, PA, US; abstract no. 396987, & EIGHTIETH ANNUAL MEETING OF THE AMERICAN ASSOCIATION FOR CANCER RESEARCH, SAN FRANSISCO, CALIF., USA vol. 30, no. 0 , 1989 , SAN FRANSISCO, USA K. HEGE ET AL. 'ANTI-TAC AND ANTI-TRANSFERRIN-TARGETED LIPOSOMES ARE EFFECTIVE FOR SPECIFIC DRUG DELIVERY TO ADULT T CELL LEUKEMIA.'
- MICKLUS ET AL: "ORGAN DISTRIBUTION OF LIPOSOMAL FORMULATIONS", BIOCHIMICA ET BIOPHYSICA ACTA 1992, , , vol. 1124, no. , pages 7 to 12
- NEUWELT ET AL: "DELIVERY OF MELANOMA ASSOCIATED IMMUNOGLOBULIN ANTIBODY AND FAB FRAGMENTS TO NORMAL BRAIN UTILIZING OSMOTIC BLOOD BRAIN BARRIER DISRUPTION", CANCER RESEARCH 1988, , , vol. 48, no. , pages 4725 to 4729

## Description

### Background of the Invention

This invention relates generally to the delivery of drugs into the brain by transcytosis across the blood-brain barrier. More particularly, the invention relates to the targeting of drug-containing liposomes to the blood-brain barrier by means of antibodies and binding fragments thereof which bind to receptors on capillary endothelial cells of the brain.

Delivery of many drugs, particularly nonlipophilic drugs, to the mammalian brain is restricted by the blood-brain barrier. The barrier is due in part to tight intercellular junctions between brain capillary endothelial cells and prevents the passive movement of many substances from the blood to the brain. The brain endothelial cells lack continuous gaps or channels connecting the luminal and abluminal membranes which would otherwise allow the passage of blood-borne molecules into the brain tissue. Instead, the presence of specific transport systems within the capillary endothelial cells, such as those for insulin, amino acids, glucose and transferrin, assure the controlled transport of compounds necessary to the functioning of the brain.

Various strategies have been developed to deliver drugs into the brain that would not otherwise be able to cross the blood-brain barrier. Commonly, although quite undesirably, an intraventricular catheter is surgically implanted to deliver a drug directly into the brain. Not only does this involve an invasive procedure that itself is potentially harmful to the patient, but the drug delivered by this means is only superficially distributed within the brain.

Other strategies have been devised to circumvent the blood-brain barrier. Some pharmacologic approaches involve chemically converting hydrophilic drugs into more lipid-soluble forms so they are more easily transported across the barrier. Another approach involves transiently opening the barrier by infusing hypertonic substances intra-arterially to allow passage of hydrophilic drugs, although hypertonic substances may be associated with toxicity and even damage the barrier.

More recently, it has been proposed to link hydrophilic neuropharmaceutical agents such as neuropeptides to other peptides which are themselves capable of crossing the blood-brain barrier by transcytosis. U.S. Patent No. 4,801,575 to Pardridge describes the preparation of chimeric peptides by coupling or conjugating the pharmaceutical agent to a transportable peptide. The chimeric peptide purportedly passes across the barrier via receptors for the transportable peptide. Transportable peptides, or vectors, mentioned as suitable for coupling to the pharmaceutical agent include insulin, transferrin, insulin-like growth factors I and II, basic albumin and prolactin. However, as noted in Pardridge et al., J. Pharm. Exp. Therap. 259:66-70 (1991), the use of insulin as a vector to the blood-brain barrier may be limited by hypoglycemia side effects and by rapid clearance of the peptide by the liver, and transferrin may be limited as a vector by its high concentration in the plasma. Friden et al., Proc. Natl. Acad. Sci. USA 88:4771-4775 (1991), have suggested that drugs conjugated to anti-transferrin receptor antibodies cross the blood-brain barrier.

It has also been suggested that liposomes can enhance drug delivery to the brain across the blood-brain barrier. See, e.g., Umezawa and Eto, Biochem. Biophys. Res. Comm. 153:1038-1044 (1988); Chan et al., Ann. Neurol. 21:540-547 (1987): Laham et al., Life Sciences 40:2011-2016 (1987); and Yagi et al., J. Appl. Biochem. 4:121-125 (1982). Liposomes are small vesicles (usually submicron-sized) comprised of one or more concentric bilayers of phospholipids separated by aqueous compartments. Although liposomes have been reported to enhance the uptake of certain drugs into the brain after intravenous injection (Chan et al., and Laham et al., ibid.), it has more recently been shown that liposomes do not cross the blood-brain barrier. Schackert et al., Select. Cancer Therapeut. 5:73-79 (1989) and Micklus et al., Biochim. Biophys. Acta 1124:7-12 (1992). As noted in Micklus et al., ibid., liposomes circulating in the plasma are ultimately taken up by the liver, digested and the lipid components released and redistributed to other organs. See also, Derksen et al., Biochim. Biophys. Acta 971:127-136 (1988). The radioactivity found in the brain following intravenous injection of labeled liposomes of Umezawa and Eto, supra, and others may in fact be derived from digested lipids and not from the intact liposomes themselves.

Many drugs, particularly neuropeptides, growth factors and the like, are rapidly degraded in the systemic circulation prior to reaching the brain, or minimally penetrate the blood-brain barrier. Many of these peptides, if effectively delivered to the brain in small quantities, could have a dramatic therapeutic effect. See, e.g., Banks and Kastin, Am. J. Physiol. 259:E1-E10 (1990). Presently, however, large amounts must be administered to allow entry of even minimal levels required for pharmacologic action.

What is needed in the art is an effective means to deliver pharmaceutical agents such as neuropeptides across the blood-brain barrier and into brain tissue. The methods and pharmaceutical compositions thereof would desirably provide uniform introduction of the pharmaceutical agent throughout the brain and present as little risk to the patient as possible. Quite surprisingly, the present invention fulfills these and other related needs.

### Summary of the Invention

The present invention provides immunoliposomes and pharmaceutical compositions thereof capable of targeting pharmacological compounds to the brain. Liposomes are coupled to an antibody binding fragment such as Fab, F(ab')₂, Fab' or a single antibody chain polypeptide which binds to a receptor molecule present on the vascular endothelial cells of the mammalian blood-brain barrier. Typically the antibody binding fragment is prepared from a monoclonal antibody. The receptor is preferably of the brain peptide transport system, such as the transferrin receptor, insulin receptor, IGF-I or IGF-2 receptor. The antibody binding fragment is preferably coupled by a covalent bond to the liposome.

Pharmacological compounds which especially benefit from this invention are those which are typically poorly transported across the blood-brain barrier, such as hydrophilic peptides, and which are often highly potent in small quantities in the brain. These include, for example, peptide neurotrophic factors, neurotransmitters and neuromodulators, e.g., beta-endorphins and enkephalins. For optimal delivery and to lessen the possibility of harmful side effects as a result of the liposome, the diameter of the liposome is less than 1 micron and typically smaller than about 0.45 microns.

A liposome containing the pharmacological compound is coupled to an antibody binding fragment which binds to a receptor molecule present on vascular endothelial cells of the mammalian blood-brain barriers, such as the transferrin receptor, insulin receptor, IGF-I or IGF-2 receptor. The pharmaceutical-containing immunoliposome is then administered to the subject in an amount sufficient to effectively treat or prevent the disorder. The preparation is usually administered parenterally, e.g., intravenously or intraarterially.

### Description of the Specific Embodiments

The present invention provides compositions for targeting drugs to and across the blood-brain barrier. The compositions, including pharmaceutical compositions, are comprised of a liposome, a pharmaceutical agent intended to be transported to the brain, and an antibody binding fragment which binds to a receptor present on vascular endothelium cells of the brain. Preferably the receptor is transferrin receptor and the antibody which binds to the receptor is a binding fragment which lacks some or all of the Fc portion of the molecule to minimize clearance of the composition by the reticuloendothelial system (RES).

Liposomes are used in the present invention to carry the pharmaceutical agent of interest to the blood-brain barrier for ultimate transport across the barrier and into the brain. As used herein and as recognized in the art, liposomes include any synthetic (i.e., not naturally occurring) structure composed of lipid bilayers that enclose a volume. Liposomes include emulsions, foams, micelles, insoluble monolayers, liquid crystals, phospholipid dispersions, lamellar layers and the like. In the liposome preparations of the invention, the pharmaceutical agent to be delivered across the blood-brain barrier is incorporated as part of a liposome in conjunction with the targeting molecule.

The receptor to which the antibody fragment/liposome is directed may be one or several receptors present in vascular endothelium of the brain. The receptor should be sufficiently accessible to be bound by the antibody fragment coupled to the liposome. Receptors which are present in higher quantities in the brain capillaries than others are preferred for enhancing levels of transport across the blood-brain barrier. Receptors which may be targeted include, for example, transferrin receptor, insulin receptor, insulin-like growth factors I and II (IGF-I and IGF-II) receptors and other receptors of the brain peptide transport system, as generally described in Pardridge, Peptide Drug Delivery to the Brain, Raven Press, New York, NY (1991), and U.S. Patent 4,801,575, the glucose transport receptor and the like.

Preferably the receptor targeted by antibody binding fragment is transferrin receptor. Transferrin receptor is selectively enriched on the endothelium of the brain microvascular endothelium of a variety of mammals, including humans, and is the primary pathway for iron to enter the brain. Iron-loaded transferrin, an 80 Kd glycoprotein, the principal iron transport protein in the circulation, undergoes transcytosis through the blood-brain barrier via the transferrin receptor. The structure and function of the transferrin receptor have been described in Seligman, Prog. Hematol. 13:131-147 (1983), which is incorporated by reference herein. Transferrin receptor is believed to project largely into the extracellular space. It can be isolated and purified from brain tissue using well known procedures. The purification, cloning, expression of human insulin receptor and the production of monoclonal antibodies thereto are described in U.S. Patent No. 4,761,371.

The targeted receptor purified from brain or expressed by recombinant DNA techniques may be used to produce antibodies, and preferably monoclonal antibodies, which may then be used to produce the antibody fragments useful in the present invention. The antibodies are made to the receptor molecule or a portion of the receptor molecule, such as that domain or domains which contributes to ligand binding. Preferably, the antibody will bind to an extracellular portion of the receptor which is proximate to the binding site of the receptor's native ligand, e.g., transferrin or insulin. In more preferred embodiments the antibody does not substantially block or compete with the binding of ligand to the receptor.

The production of monoclonal antibodies to transferrin receptor, including the OX-26 monoclonal antibody described in the Examples below, has been described in Jeffries et al., Immunology 54:333-341 (1985), which is incorporated herein by reference. Other monoclonal antibodies to transferrin receptor have been described. See, e.g., the ATCC Catalogue of Cell Lines and Hybridomas, 7th ed., 1992, describing HB21, a murine IgG1 antibody which precipitates human transferrin receptor; HB84, a murine IgG2a monoclonal antibody to human transferrin receptor; and TIB219 and TIB220, rat anti-murine transferrin receptor antibodies. Monoclonal antibodies to the human insulin receptor have also been reported, including cell lines available from the ATCC, such as ATCC HB175 (J. Biol. Chem. 258:6561-6566 (1983)) and ATCC CRL 1827 (Curr. Top. Cell. Reg. 27:83-94 (1985)).

Methods for the production of monoclonal antibodies are well known and may be accomplished by, for example, immunizing the animal with cells which express the receptor, substantially purified receptor, or fragments thereof as discussed above. Antibody producing cells obtained from immunized animals are immortalized and screened, or screened first for the production of antibody which binds to the receptor protein and then immortalized.

As an antibody fragment used in the present invention will generally lack the immunogenic Fc portion, the necessity of using human monoclonal antibodies is substantially avoided. However, it may be desirable to transfer antigen binding regions of the non-human antibodies, e.g., the hypervariable regions, to human framework regions by recombinant DNA techniques to produce substantially human Fab' molecules. Such methods are generally known in the art and are described in, for example, EPO publications 173,494 and 239,400, which are incorporated herein by reference. The production of single polypeptide chain binding molecules, also referred to as single chain antibodies, by recombinant DNA techniques is described in detail in U.S. Patent No. 4,946,778.

The antibody to the brain receptor molecule may be used intact or, more preferably, as a binding fragment thereof. By binding fragment is meant that the fragment retains an ability to specifically bind to an epitope of the target molecule of interest, such as transferrin or insulin receptor, for example. Antibody binding fragments include at least the hypervariable or complementarity determining region (CDR) situated in an appropriate framework to produce a conformation which binds to the antigenic determinant. These binding fragments include, but are not limited to, Fv, Fab, F(ab')₂, Fab', and single polypeptide chain binding molecules.

Antibody binding fragments can be produced from intact antibody molecules by a variety of procedures well known to those of skill in the art. For example, antibodies are usually fragmented by partial digestion with papain to produce two Fab fragments. Pepsin treatment can be used to produce the F(ab')₂ fragments where the two antigen binding domains are still bound together, i.e., as a multivalent binding molecule. Further reduction of the F(ab')₂ fragment with, e.g., dithiothreitol can be used to separate the antigen binding domains and produce two F(ab') fragments. The preparation of the various antibody fragments is described in detail in, e.g., Harlow and Lane, Antibodies, A Laboratory Manual, Cold Spring Harbor Laboratory Press, Cold Spring Harbor, NY 1988 pp. 626-631, and Parham, in Cellular Immunology, 4th ed., (ed. D.M. Weir), vol. 1, chap. 14, Blackwell Scientific Publ., CA 1986. The polypeptide chains of the desired fragments may also be produced by a variety of recombinant DNA techniques and assembled intra- or extracellularly.

Once the desired antibody or, more preferably, an antibody binding fragment, has been obtained it is coupled to or incorporated into the liposome. Liposomes for use in the invention may be formed from standard vesicle-forming lipids, which generally include neutral and negatively charged phospholipids and a sterol, such as cholesterol. The selection of lipids is generally guided by consideration of, e.g., liposome size and stability in the bloodstream. Thus, the lipids which can be employed in the present invention include, e.g., cholesterol hemisuccinate and salts thereof, tocopherol hemisuccinate and salts thereof, a glycolipid, a phospholipid such as phosphatidylcholine, phosphatidylethanolamine (PE), phosphatidylserine, phosphatidylglycerol, phosphatidic acid, phosphatidylinositiol, sphingomyelin, and the like, alone or in combination. The phospholipids can be synthetic or derived from natural sources such as egg or soy. Phosphatidylcholines having a variety of acyl chain groups of varying chain length and degree of saturation are available or may be isolated or synthesized by well known techniques. In general, less saturated phosphatidylcholines are more easily sized, particularly when the liposomes are sized below about 0.3 microns. The acyl chain compositions of phospholipid may also affect the stability of liposomes in the blood. Sterols such as cholesterol can be combined with the phospholipids. The phospholipids employed and the amount of sterol present depends on a number of factors such as lipophilicity of any added pharmaceutical agent, the targeting antibody fragment, and required properties of the liposome. These factors are generally known to those skilled in the art.

A wide variety of methods for preparing liposomes suitable for targeting to the blood-brain barrier in the present invention are available. See, for example, Szoka et al., Ann. Rev. Biophys. Bioeng. 9:467 (1980), Liposome Technology, ed. G. Gregoriadis, CRC Press, Inc., Boca Raton, FL (1984), U.S. Patent Nos. 4,235,871, 4,501,728, 4,837,028 4,957,735 and 5,019,369. Since the targeted liposomes of the invention are not intended for uptake by the reticuloendothelial system, the lipid components are further selected to increase time in the bloodstream.

Following liposome preparation, the liposomes may be sized to achieve a desired size range and relatively narrow distribution of liposome sizes. For delivery to the brain, liposomes should generally be less than about 1.0 microns in size, more preferably about 0.2 to 0.45 microns, which allows the liposome suspension to be sterilized by filtration. For sizing liposomes, a liposome suspension may be sonicated either by bath or probe down to small vesicles of less than about 0.05 microns in size. Homogenization may also be used, which relies on shearing energy to fragment large liposomes into smaller ones. Homogenizers which may be conveniently used include microfluidizers produced by Microfluidics of Boston, MA. In a typical homogenization procedure, liposomes are recirculated through a standard emulsion homogenizer until selected liposomes sizes, typically between 0.1 and 0.5 microns, are observed. In both methods the particle size distribution can be monitored by conventional laser-beam particle size discrimination.

Extrusion of liposomes through a small-pore polycarbonate membrane or an asymmetric ceramic membrane is an effective method for reducing liposome sizes to a relatively well defined size distribution. Typically, the suspension is cycled through the membrane one or more times until the desired liposome size distribution is achieved. The liposomes may be extruded through successively smaller pore membranes to achieve a gradual reduction in liposome size.

Methods for coupling antibodies to liposomes generally involve either covalent crosslinking between a liposomal lipid and a native or modified antibody (or binding fragment thereof). In another approach, an antibody which has been covalently derivatized with a hydrophobic anchor, such as a fatty acid, is incorporated into a preformed lipid.

A variety of crosslinking agents can be employed to produce a covalent link between a lipid and the antibody. One protocol involves the derivatization of the free amino group of a PE with an amino reactive bifunctional crosslinker. The derivatized PE along with other lipids are then used to form liposomes by the methods described above. Once incorporated into the liposomes the derivatized PE can be reacted with the antibody by using the second reactive site on the crosslinking reagent. The use of heterobifunctional reagents are particularly useful because homocoupling between liposomes or between antibodies is avoided. Heterobifunctional crosslinkers which can be used include N-hydroxysuccinimidyl 3-(2-pyridythio) propionate (SPDP), m-maleimidobenzoyl-N-hydroxysuccinimde (MBS) and the like, and homobifunctional crosslinkers include toluene-2,4-diisocyanate (TDIC) and 1-ethyl-3-(3-dimethylaminopropyl) carbodiimide (EDCI)). Chemical crosslinking techniques also include the use of glutaraldehyde. These and other regents are reviewed in Connor and Huang, Pharmac. Ther. 28:341-365 (1985).

The covalent attachment of antibody fragments to liposomes by crosslinking provides the advantage of attaching a large amount of antibody binding fragment to the liposome surface, with little or no loss of entrapped aqueous content using these methods. Care should be taken, however, that the crosslinking conditions are not so harsh that antibody binding is adversely affected, or that the liposomes and their contents are damaged. To avoid the orientation of antibody molecules which may render them incapable of binding to antigen, a spacer arm between the antibody and the lipid may optionally be employed.

A non-crosslinking association of antibodies or binding fragments thereof with liposomes can also be employed. This approach allows more than one type of antibody binding specificity per liposome, e.g., antibodies or fragments to different antigenic determinants on the transferrin molecule or even antibodies which bind to different receptors. The flexibility of the molecule minimizes steric hindrance which may block binding of the antibody to the antigen. Also, the reagents involved in the non-covalent methods are relatively mild and do not come into direct contact with the contents of the liposomes, thus avoiding possible damage to the liposome components.

In one method the non-covalent association of the antibody or fragment with the liposome can be accomplished by derivatizing the antibody component with a hydrophobic group. The derivatized antibody is then incorporated into the vesicles by inserting the hydrophobic anchor into the bilayer. Methods of derivatization include reacting the antibody with the N-hydroxysuccinimide ester of palmitic acid (NHSP) in the presence of 1-2% detergent, typically deoxycholate, as described in Huang et al., Biochem. Biophys. Acta 716:140-150 (1982). Alternatively, palmitic acid chloranhydride can replace NHSP as the acylation reagent.

Another method to acylate the antibody involves derivatizing the head group of a PE or similar molecule rendering it capable of reacting with a sulfhydryl group. The antibody's disulfide bonds are then reduced with dithiothreitol (DTT) and then incubated with the derivatized PE. In yet another method useful in the present invention, a free PE or similar molecule is crosslinked to the carboxylic groups of an antibody by carbodiimide as described in Jansons and Mallett, Analyt. Biochem. 11:54-59 (1981), protecting the free amino groups of the antibody prior to the crosslinking to prevent homocoupling between antibody molecules.

Once derivatized, the antibody or binding fragment thereof can become associated into the liposomes by a variety of established protocols. For example, the derivatized antibody is mixed with the liposome in the presence of detergent, and the detergent then removed by dialysis to form the immunoliposomes. Another method involves a modification of the reverse-phase evaporation vesicle preparation method, where the preformed vesicles are mixed with antibody (lipid:antibody = 10:1 w/w) in a deoxycholate solution in the presence of ether, and both the detergent and ether are subsequently removed by extensive dialysis. See, e.g., Huang et al., in Meths. Enzymol. (1985). The preparation of immunoliposomes is also described in U.S. Patent No. 4,957,735. Preferably the antibody or binding fragment thereof is attached to the liposome component after the liposome is prepared.

A variety of drugs or other pharmacologically active agents are suitable for incorporation into the liposome and thus delivery to the blood-brain barrier. The use of liposomes as drug delivery vehicles is extensively reviewed in Gregoriadis, (ed.), Liposomes as Drug Carriers: Recent Trends and Progress, John Wiley & Sons, NY (1988). Therapeutic agents which may be delivered include protein neurotrophic factors, e.g., nerve growth factor, to treat brain injury and degenerative diseases, enzymes to replace those lost through genetic defects causing metabolic storage diseases, e.g., Tay-Sachs disease, neurotransmitters and neuromodulators, e.g., dopamine and beta-endorphin for treating Parkinson's disease, conditions associated with pain, disorders of movement or cognition and behavior, antibiotics, chemotherapeutic agents, diagnostic imaging agents, etc. Particularly useful in the present invention are drugs such as peptides which have specific effects in the brain but which poorly cross into the brain normally, and have no or little effect in other organs. These compounds are often readily broken down in the bloodstream, and thus benefit from the protection provided by encapsulation in a liposome of the present invention. When targeted to a brain transport system as described herein, even a small amount crossing into the brain can provide a desired pharmacological effect.

One general class of drugs include water-soluble, liposome-permeable compounds which are characterized by a tendency to partition preferentially into the aqueous compartments of the liposome suspension, and to equilibrate, over time, between the inner liposomal spaces and outer bulk phase of the suspension. Representative drugs in this class include propranolol, ibuprofin, gentamicin, tobramycin, penicillin, theophylline, bleomycin, etoposide, n-acetyl cysteine, verapamil, vitamins, and radio-opaque and particle-emitter agents, such as chelated metals. Because of the tendency of these agents of equilibrate with the aqueous composition of the medium, it is preferred to store the liposome composition in lyophilized form, with rehydration shortly before administration. Alternatively, the composition may be prepared in concentrated form, and diluted shortly before administration.

A second general class of drugs are those which are water-soluble, but liposome-impermeable. For the most part, these are peptide or protein molecules, such as peptide hormones, enzymes, enzyme inhibitors, apolipoproteins, and higher molecular weight carbohydrates characterized by long-term stability of encapsulation. Representative compounds in this class include nerve growth factor, interferon (α, β or γ), oxytocin, vasopressin, insulin, interleukins (e.g., IL-1, IL-2, etc.), superoxide dismutase, tumor necrosis factor, somatostatin, thyrotropin releasing hormone, and macrophage colony stimulating factor, among others. Peptide molecules and hormones which are typically very potent, and thus even small amounts crossing the blood-brain barrier can affect function, are particularly useful in targeted methods and compositions of the present invention. They include, for example, the β-endorphins and analogues, the enkephalins (relatively lipid soluble) such as Leu- and Met-enkephalins and analogues, melanocyte-stimulating hormone and melanocyte-stimulating hormone inhibitory factor, β-casomorphin, glucagon, delta sleep-inducing peptide and others, some of which are discussed in Banks and Kastin, supra,

A third class of drugs are lipophilic molecules which tend to partition into the lipid bilayer phase of the liposome, and which are therefore associated with the liposomes predominantly in a membrane-entrapped form. The drugs in this class are defined by an oil/water partition coefficient, as measured in a standard oil/water mixture such as octanol/water, of greater than 1 and preferably greater than about 5. Representative drugs include prostaglandins, amphotericin B, methotrexate, cis-platin and derivatives, vincristine, vinblastine, progesterone, testosterone, estradlol, doxorubicin, epirubicin, beclomethasone and esters, vitamin E, cortisone, dexamethasone and esters, betamethasone valerete and other steroids, etc.

Following treatment to remove tree drug and/or targeting antibody molecule, the liposome suspension is brought to a desired concentration in a pharmaceutically acceptable carrier for administration to the patient. The pharmaceutical compositions are intended for parenteral, topical, oral or local administration, but preferably they are administered parenterally, e.g., intravenously, intraarterially or intramuscularly. Thus, this invention provides compositions for parenteral administration targeted for the blood-brain barrier which comprise a solution of a selected pharmaceutical contained in liposome associated with an antibody molecule or binding fragment thereof which binds to a brain receptor molecule, preferably the transferrin receptor molecule, dissolved in a pharmaceutically acceptable carrier, preferably an aqueous carrier. A variety of aqueous carriers may be used, e.g., water, buffered water, 0.4% saline, 0.3% glycine, and the like, including glycoproteins for enhanced stability, such as albumin, lipoprotein, globulin, etc. These compositions may be sterilized techniques referred to above or produced under sterile conditions. The resulting aqueous solutions may be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such an pH adjusting and buffering agents, tonicity adjusting agents and the like, for example, sodium acetate, sodium lactate, sodium chloride, potassium chloride, calcium chloride, etc.

The concentration of the liposomes in these formulations can vary widely, i.e., from less than about 0.5%, usually at or at least about 1% to as much as 15 or 20% by weight and will be selected primarily by fluid volumes, viscosities, etc., in accordance with the particular mode of administration selected. Actual methods for preparing parenterally administrable liposomes formulations will be known or apparent to those skilled in the art and are described in detail in, for example, Remington's Pharmaceutical Science, 17th ed., Mack Publishing Company, Easton, PA (1985), and Gregoriadis (1988), supra.

The dose and the route of administration and the carrier used may vary based on the disease being treated and in view of known treatments for such diseases. Amounts effective for a particular use will depend on the severity of the disease and the weight and general state of the patient being treated. It must be kept in Mind that the materials of the present invention may be employed in serious disease states, that is, life-threatening or potentially life threatening situations. In such cases, in view of the ability of the liposomes to target the blood-brain barrier and the minimal immunogenicity of the antibody binding fragments, it is possible and may be felt desirable by the treating physician to administer substantial excesses of these compositions.

The following examples are provided by way of illustration, not limitation.

### EXAMPLE I

### Production of Anti-Transferrin Receptor Fab'-Targeted Immunoliposomes

This Example describes the production of Fab'-coupled immunoliposomes for targeting to the transferrin receptor.

### A. Preparation of Liposomes

Liposomes were prepared with a Heat Systems W 380 cup horn sonicator (Farmington, NY) by a method modified from Barrow and Lentz, Biochim. Biophys. Acta, 597:92-99 (1980). L-a-Phosphatidylcholine (PC) and N-[4-p-malimido phenyl butyryl] dioleoyl phosphatidylethanolamine (MPB-PE) were obtained from Avanti Polar Lipids, Inc. (Pelham, AL), and cholesterol (Chol) was purchased from Sigma Chemical Co. (St. Louis, MO). Aliquots of chloroform (or chloroform/methanol) solution containing the appropriate lipids (PC/Chol/MPB-PE lipids at a molar ratio of 10/5/1), were pipetted into 5-ml glass test tubes. [¹⁴C]-labeled dipalmitoylphosphatidylcholine (DPPC) (purchased from Amersham, Arlington Heights, IL), was added to give a final aqueous concentration of 0.5 µCi/ml. Solvents were evaporated under nitrogen, and solvent traces were removed under vacuum. Lipid films were dispersed in 0.9% (w/v) saline (or Hopes-buffered 0.9% saline), transferred to 10 ml polycarbonate test tubes in 1-2 ml aliquots, and sonicated continuously at 50°C for 2-3 min to visual clarity. Liposomes were checked for diameter site by filtration through 0.45 micron-diameter cellulose acetate filters (Millipore, Boston, MA).

### B. Preparation of Antibody Fragments

F(ab')₂ fragments were prepared from "OX-26" anti-transferrin receptor monoclonal antibody (purchased from Bioproducts for Science, Inc. (Indianapolis, IN) by Loftrand Lab LTD (Gaithersburg, MD). Nonspecific rabbit IgG F(ab')₂ was purchased from Organon Teknika-Cappel (Durham, NC). Fab' fragments were generated from OX-26 F(ab')₂ (10 mg/ml) and nonspecific rabbit F(ab')₂ fragments (10 mg/ml) by reduction with 20 mM DTT (Sigma) in 20 mM HEPES (pH 6.5) for 60 min. Fab' fragments were separated from excess DTT on minicolumns of Sephadex G-25 (Pharmacia Fine Chemicals, Uppsala, Sweden), made from the barrels of 3-ml plastic syringes according to the technique of Fry et al., Anal. Biochem. 90:809-815 (1987). One-half ml aliquots of reduced Fab' were placed on previously centrifuged minicolumns and centrifuged at 1000 rpm for 10 min. The degree of reduction was determined by the method of Ellman, Arch. Biochem. Biophys. 82:70-77 (1959).

### C. Coupling of Fab' Fragments to Liposomes

Freshly reduced Fab' fragments were added to equivalent volumes of freshly prepared liposomes (in 20 mM Hopes-buffered saline, pH 6.5) and mechanically stirred at room temperature overnight.

Fab'-coupled liposomes were isolated from the reaction mixture by flotation on a metrizamide (Sigma) gradient by the method of Heath et al., Biochim. Biophys. Acta 640:66-81 (1981). Briefly, 1 ml of liposomal suspension was mixed with 1 ml of 1 M metrizamide solution in 0.9% saline in a 5 ml nitrocellulose centrifuge tube. This solution was gently overlaid with 3 ml of 0.3 M metrizamide in 0.9% saline and topped with 0.5 ml of 0.9% saline. The solution was centrifuged at 40,000 rpm for 1 h in a L8-80M ultracentrifuge (Beckman Instruments, Palo Alto, CA). The liposomes were pipetted off the gradient, and the metrizamide was removed on a Sephadex G-25 minicolumn as previously described.

The protein concentrations of OX-26 anti-transferrin IgG Fab', and of nonspecific rabbit IgG Fab' (control) associated with the related liposomal formulations were estimated colorimetrically (Sigma Protein Assay Kit) to be 60 and 140 µg/ml, respectively.

Fab'-coupled liposomal formulations were not tested for receptor site binding. However, conjugates prepared from SATA-modified proteins have been shown to retain enzyme activity or function (Duncan et al., Anal. Biochem. 132:68-73 (1983)).

### EXAMPLE II

### Localization in the Brain of Immunoliposomes Targeted to Transferrin Receptor

This Example demonstrates that liposomes covalently coupled to Fab' fragments can bind to transferrin receptors in brain capillaries. Rats were used in these studies to demonstrate localization of the antibody fragment-targeted liposomes. Rats have a brain capillary system that, although smaller overall than the capillary system found in a human brain, is not substantially different. Further, radiolabeled OX-26 monoclonal antibody has been shown to bind isolated brain capillaries from rats and humans to approximately the same extent. Pardridge et al., J. Pharmacol. Exp. Ther. 259:66-70 (1991).

### A. Intracarotid Infusion in Rats

Three-month-old male Sprague-Dawley rats (Charles River Breeding Laboratories, Lakeview, NJ) weighing approximately 200-300 g were given free access to food and water and acclimated for at least 2 days to the Laboratory of Neurosciences animal room, where temperature, humidity and light cycles (6-18 h) were controlled. Experiments were conducted consistent with NIH guidelines, under a protocol approved by the NICHD Animal Care and Use Committee.

Intraarterial liposomal administration was undertaken to maximize liposome delivery and binding to the ipsilateral cerebral vasculature. The advantage of this administration route occurs only during the initial passage of liposomes through ipsilateral brain. Liposomes then enter the systemic circulation and show pharmacokinetic behavior similar to liposomes administered intravenously (Greig, N. in Implications of the Blood-Brain Barrier and its Manipulation, vol. 1: "Basic Science Aspects," (Neuwelt, E., ed.), pp. 311-368, Plenum Press, New York, NY (1989).

Rats anesthetized by i.p. injection of 50 mg/kg Na-pentobarbital (Ayerst, New York, NY) had sodium heparin-filled (100 I.U. in isotonic saline) catheters (size PE-50) inserted into the right external carotid and femoral arteries, according to the procedure of Rapoport et al., Am. J. Physiol. 238:R421-R431 (1980).

Liposomal formulations containing trace levels (0.5 µCi/ml) of ¹⁴C]-labeled DPPC were administered by infusion into the carotid artery catheter at a rate of 0.2 ml/min for 5 min (infusion pump No. 944, Harvard Instruments, Millis, MA). The carotid bifurcation was observed to ensure that the infusate passed cephalad into the internal carotid artery and not in a retrograde fashion down the common carotid artery. By linking the carotid catheter to a pressure transducer (Statham Instruments, Oxnard, CA), carotid artery pressure was monitored to ensure that hypertensive blood-brain barrier opening did not occur. After 1 or 10 min, the rats were killed by decapitation, and brain, kidneys, liver, and spleen were removed from each animal and were weighed. Plasma, brain (cerebrum ipsilateral and contralateral to infusion site, and cerebellum), other organ samples, and aliquots of liposomal formulations were measured for radioactivity (Tri-Carb 2200 CA Liquid Scintillation Analyzer, Packard Instruments, Downers Grove, IL) according to the technique of Greig et al., J. Pharmacol. Exp. Ther. 245:1-7 (1988).

### B. Calculations

Radioactivity in organ samples was calculated as mean DPM/total DPM injected. Volumes of distribution (V_{d}) for [¹⁴C]-DPPC-labeled liposomal formulations were calculated as follows: V_{d}-DPM per gram wet tissue weight/DPM per ml plasma x 100 (% wet wt). In the calculation of radioactivity distributions, blood volume was assumed to be 6.5% of the total body weight (Altman, Blood and Other Body Fluids, Biological Handbook Series, Fed. Amer. Soc. Exp. Biol., Wash., D.C. (1961)). Tissue counts were corrected for counts present in residual blood (0.01 in brain, 0.1 in spleen, kidneys, and liver (ibid.).

Assuming the liposomal diameter to be approximately 1000 Å, the lipid bilayer thickness 37 Å (Mason and Huang, Ann. N.Y. Acad. Sci. 308:29-48 (1978)), and the lipid (liposomal) density 0.9 g/cm³, the number of liposomes bound to the blood-brain barrier and the surface area occupied by bound liposomes were estimated. Capillary surface area in rat brain was estimated as 135 cm²/g wet weight brain (Altman, supra).

Dunnett's test was carried out to compare two means (Miller, R. Simultaneous Statistical Inferences pp. 67-87, McGraw-Hill, New York, Ny (1966)). A paired *t*-test was used to compare the perfused with unperfused sides of the brain of each rat. The value for statistical significance in all tests was P < 0.05.

The organ distributions of [¹⁴C]-DPPC-labeled liposomal formulations following right-sided carotid infusion in 10 rats for each study are summarized In Table I. OX-26 liposomes shoved a 50% greater association with brain than the corresponding nonspecific Fab' controls at 1 and 10 min. Levels of radioactivity in brain of rats infused with OX-26 liposomes were statistically greater than in controls at 1 min (P < 0.05, Dunnett's test) but not at 10 min. Although brain levels were essentially unchanged at 10 min, statistical significance was not achieved because of greater variability associated with the data at 10 min.

Left-right differences in cerebrum distribution values in individual animals were assessed by paired t-test. Although values for cerebrum ipsilateral to infusion (right) were greater than those of contralateral (left) cerebrum, these did not reach statistical significance. OX-26 and OX-26 control liposomes had similar liver uptakes; however, the spleen uptake of OX-26 liposomes was approximately 300% of controls at 1 and 10 min. OX-26 liposomes cleared from plasma more rapidly than controls. There was insignificant uptake of all liposomal formulations by kidney. Recoveries of all liposomal formulations averaged 75 ± 4% (SEM) of the injected dose.

V_{d} values, radioactivity per g wet weight of each organ divided by radioactivity per ml of plasma, normalize variability in plasma pharmacokinetics and liposomal plasma clearances among rats. The V_{d} values of the liposomal formulations (Table II) for OX-26 in brain were significantly greater than their respective controls at both 1 and 10 min, by approximately 2-fold at 1 min and by 6-8-fold at 10 min (p < 0.05, Dunnett's test). Although the V_{d} value for right cerebrum was up to 3.5 fold greater than left cerebrum, this did not reach statistical significance (paired t-test). At 1 and 10 min, high levels of OX-26 liposomes were present in liver and spleen, indicating liposomal accumulation in these organs. V_{d} values of all formulations in brain tissue did not increase between 1 and 10 min, indicating that liposomes did not measurably cross the blood-brain barrier.

The foregoing results indicate that liposomes covalently coupled to antibody binding fragments, and particularly Fab' fragments can bind to transferrin receptors in brain capillaries. Bound liposomes were estimated to be approximately 0.03% of the injected dose or 0.025 pmol of liposomes. Control liposomes associated with rat brain were subtracted from total brain uptake of OX-26 liposomes because brain radioactivity levels are slightly elevated (above background) after intracarotid infusion (Micklus et al., Biochim. Biophys. Acta 1124:7-12 (1992)). Transferrin receptor density in rat brain has been estimated to be about 0.02 pmole per gram of brain (Pardridge et al., J. Pharmacol. Exp. Ther. 259:66-70 (1991)), generally equivalent to 0.034 pmole per brain for the rats used in the present experiments, or approximately 1 liposome per 1.3 receptors. OX-26 liposomal brain uptake remained constant rather than increasing after 1 min, suggesting that essentially all the receptor sites were occupied after 1 min. This result is consistent with the observation that OX-26 antibodies show significant binding to capillaries at 5 min after injection (Friden et al., Proc. Natl. Acad. Sci. USA 88:4771-4775 (1991)). No increased brain liposomal uptake was observed during the brief 10 min period used in this study.

Comparison of intra-animal left to right cerebrum distributions and V_{d} values of our liposomal preparations showed no statistical difference, indicating that receptor sites are rapidly saturated in both cerebral hemispheres. This suggests that similar receptor site saturation could be achieved by (1) intravenous administration of liposomes and/or (2) a reduction in the liposomal dose.

The capillary surface area in rat brain cortex is about 135 cm² per gram of brain (wet wt), or 230 cm² for rats in the present study; therefore, it was estimated that about 0.7% of brain capillary surface was covered with liposomes. Liposomal binding to brain can be increased to cover all of the capillary surface by upregulating the transferrin receptor or targeting other receptors, e.g., in a 270 g rat as used in this study, this would represent 3.8 pmol of liposomal brain uptake.

Transport of the Fab'-targeted liposome across the blood-brain barrier is demonstrated in experiments performed according to the brain capillary depletion procedure as described in Pardridge et al., J. Pharmacol. Exp. Therap. 259:66-70 (1991), and Triguero et al., J. Neurochem. 54:1882-1888 (1990).

In other aspects, OX-26 Fab'-coupled liposomes showed significant liver and spleen uptakes at 1 and 10 min after infusion. OX-26 liposomes demonstrated an unusually high spleen uptake, with 6% of their total radioactivity present in the spleen after 1 min, and 12% after 10 min. This enhanced uptake is likely due to the many transferrin receptors in spleen or on red blood cells, which are rapidly cleared by the spleen after binding to OX-26 liposomes. This suggests that OX-26 liposomes can be used to effectively target therapeutic agents to the spleen.

### EXAMPLE III

### Liposomes Targeted to Insulin Receptor

This Example compares the ability of insulin-targeted liposomes to distribute labeled formulations in brain when compared to control preparations. Although the brain distribution of insulin-coupled liposomes was not statistically different from the control formulations, V_{d} values, which adjust data for plasma clearance, were higher for insulin liposomes in right cerebrum than controls at 1 and 10 min, suggesting that a moderate binding to brain capillaries does occur.

### Formulations

The [¹⁴C]-DPPC-labeled liposomal formulations evaluated in this study consisted of 8 µmol/ml suspensions of PC/Chol/MPB-PE lipids (molar ratio, 10/5/1), covalently coupled to proteins. The protein concentrations of insulin, of OX-26 anti-transferrin IgG Fab', and of nonspecific rabbit IgG Fab' (control) associated with the related liposomal formulations were estimated colorimetrically (Sigma Protein Assay Kit) to be 250, 60 and 140 µg/ml, respectively.

### A. Preparation of Thiolated Insulin

Insulin (porcine, sodium) and N-succinimidyl-S-acetyl-thioacetate (SATA) and were obtained from Cal Biochem Corp. (LaJolla, CA). Insulin was thiolated with SATA by a modification of the method of Duncan et al., Anal. Biochem 132:68-73 (1983). Twenty µl of SATA in dimethyl formamide (DMF) (100 mg/ml) was reacted per 1 ml of insulin solution (10 mg/ml insulin + 5 µl 5 N NaOH) in deionized water for 30 min. Thioacetylated insulin was deacetylated by reaction with 100 µl (per ml insulin solution) of aqueous 0.5 M hydroxylamine (pH 6.8) for 30 min. Thiolated insulin was separated from low molecular weight solutes on minicolumns of Sephadex® G-25, made from the barrels of 3-ml plastic syringes according to the technique of Fry et al., Anal. Biochem. 90:809-815 (1978). One-half ml aliquots of thiolated insulin solution were placed on previously centrifuged minicolumns and centrifuged at 1,000 rpm for 10 min. After addition of 0.2 ml of saline, this centrifugation stop was repeated. The extent of thiolation was determined by the method of Ellman, supra.

### B. Coupling of Insulin to Liposomes

Freshly thiolated insulin was added to an equivalent volume of freshly prepared liposomes (in 20 mM Hepes-buffered saline, pH 7.5) and mechanically stirred at room temperature overnight. Insulin-coupled liposomes were isolated from the reaction mixture by flotation on a metrizamide gradient as described in Example I above.

The protein concentration of insulin associated with the liposomal formulation was estimated colorimetrically to be 250 µg/ml. Insulin-coupled liposomal formulations were not tested for receptor site binding. However, conjugates prepared from SATA-modified proteins have been shown to retain enzyme activity or function (Duncan et al., supra).

Animal studies were performed in accordance with the procedures described in Example II above.

The results, shown in Table I and Table II, indicate that insulin and insulin control liposomes had comparable distributions in brain and, similarly, there were no significant right-left cerebrum differences following administration of either preparation. Insulin liposomes cleared from plasma more rapidly than control liposomes and had greater liver and spleen uptakes.

V_{d} values of insulin liposomes in right cerebrum were significantly higher than control values at 1 and 10 min by approximately 2-fold (P < 0.05, Dunnett's test). Values in left cerebrum, however, were not different from controls, nor were there left-right differences in cerebrum V_{d} values.

The brain distribution of insulin-coupled liposomes was not statistically different from the control formulations, suggesting that they do not significantly bind to rat brain capillary. However, V_{d} values, which adjust data for plasma clearance, for insulin liposomes in right cerebrum (ipsilateral to liposome infusion) were higher than controls at 1 and 10 min, suggesting that a moderate binding to brain capillaries does occur. Possible reasons for such moderate binding are (1) low insulin binding affinity between liposome and capillary receptor (2) partial inactivation of the insulin during the liposomal coupling reaction, (3) low insulin receptor density at the cerebral capillary, and (4) competition between insulin liposomes with endogenous insulin for brain capillary receptor sites.

### EXAMPLE IV

### Liposomes Targeted Via Antibody Fragments to Insulin Receptor

A liposome is prepared according to Example I, incorporating a transportable neuropeptide to be targeted to the brain of an animal. A monoclonal antibody to human insulin receptor is digested to prepare Fab' fragments which are coupled to the liposome as described in Example I. The resulting targeting immunoliposome is combined with sterile saline to provide a physiologically acceptable solution, and is administered to the patient or subject parenterally.

### EXAMPLE V

### Liposomes Targeted Via Antibody Fragments to IGF-I Receptor

A liposome is prepared according to Example I, incorporating a pharmacological compound to be targeted for delivery to the brain of a patient or subject. A monoclonal antibody to human IGF-I receptor is digested as described in Example I to prepare Fab' fragments. The Fab' fragment are coupled to the liposome as described in Example I. The resulting targeting immunoliposome is combined with sterile saline to provide a physiologically acceptable solution, and is administered to the patient or subject parenterally.

**Table I**

| Distribution of [¹⁴C]-DPPC-labeled Liposomal Formulations in Rats: % DPM Recovered in Organs 1 and 10 Min after Intracarotid Injection.^{a} | | | | | | | | |
|---|---|---|---|---|---|---|---|---|
| Min | Formulation | Brain | Cerebrum/Right | Cerebrum/Left | Cerebellum | Spleen | Liver | Plasma |
| 1 | OX-26 Fab' | .09±.01* | .05±.01* | .015±.002* | .03±.003* | 6.1±.7* | 14±1 | 34±4 |
| | OX-26 Control | .06±.01 | .02±.002 | .01±.001 | .02±.002* | 1.9±.3 | 23±6 | 46±7 |
| | Insulin | .05±.01 | .03±.01 | .01±.01 | .01±.002* | 2.4±.2* | 31±4* | 50±6* |
| | Insulin Control | .05±.002 | .02±.003 | .013±.002 | .02±.002 | 0.8±.1 | 5±.6 | 73±3 |
| 10 | OX-26 Fab' | .09±.02 | .05±.01 | .03±.01 | .02±.004 | 11±2* | 27±3 | 24±5* |
| | OX-26 Control | .06±.01 | .03±.01 | .01±.002 | .02±.004 | 3.0±.7 | 20±4 | 63±6 |
| | Insulin | .05±.01 | .03±.004 | .015±.003 | .01±.001* | 3.3±.3* | 34±5* | 45±5* |
| | Insulin Control | .06±.005 | .02±.003 | .02±.002 | .02±.002 | 1.4±.1 | 10±1 | 67±5 |

| | | | | | | | | |
|---|---|---|---|---|---|---|---|---|
| ^{a} Data represent mean ± SEM (n = 10). OX-26 control is nonspecific rabbit IgG Fab'. Analysis of individual differences in right/left cerebrum values showed no significant difference, where P<0.05 (paired t test). | | | | | | | | |
| * Denotes statistical difference from control value, where P<0.05 (Dunnett's test). | | | | | | | | |

**Table II**

| V_{d} Values for [¹⁴C]-DPPC-labeled Liposomal Formulations in Rats 1 and 10 Min after Intracarotid Injection.^{a} | | | | | | |
|---|---|---|---|---|---|---|
| Min | Formulation | Cerebrum/Right | Cerebrum/Left | Cerebellum | Spleen | Liver |
| 1 | OX-26 Fab | 2.6±.5* | 0.7±0.1* | 2.5±0.4* | 220±50* | 47±7 |
| | Control Fab' | 0.9±0.2 | 0.4±0.1 | 1.2±0.2 | 70±10 | 75±22 |
| | Insulin | 0.8±0.1* | 0.5±0.1 | 0.9±0.1 | 74±12* | 61±12* |
| | Insulin Control | 0.4±0.1 | 0.3±0.03 | 0.9±0.1 | 16±3 | 7±1 |
| 10 | OX-26 Fab' | 4.0±1.3* | 2.5±1.0* | 5.3±1.5* | 1080±370* | 200±60* |
| | Control Fab' | 0.6±0.2 | 0.3±1.0 | 1.1±0.2 | 132±14 | 45±14 |
| | Insulin | 0.9±0.1* | 0.5±0.1 | 1.0±0.1 | 130±20* | 79±6* |
| | Insulin Control | 0.5±0.05 | 0.4±0.05 | 1.0±0.1 | 28±3 | 16±2 |

| | | | | | | |
|---|---|---|---|---|---|---|
| ^{a} Data represent mean ± SEM (n = 10). OX-26 control is nonspecific rabbit IgG Fab'. Analysis of individual differences in right/left cerebrum values showed no significant difference, where P<0.05 (paired t test). | | | | | | |
| * Denotes statistical difference from control value, where P<0.05 (Dunnett's test). | | | | | | |

It is evident from the above results that the subject invention provides antibody binding fragment-targeted liposomal formulations useful in the delivery of therapeutics and diagnostic compounds across the blood-brain barrier. In particular, the presence of antibody binding fragments in a liposome which are specific for brain transport receptor, such as transferrin receptor, insulin receptor, IGF-I or IGF-II receptor provides an effective means to enhance the uptake of certain drugs, such as hydrophilic drugs, peptides and proteins to the brain. The antibody binding fragment-modified liposomal formulations may be produced by a variety of means. Thus, increased efficacy, convenience and economics of lower dosages and less frequent administration to achieve therapeutic levels of drugs in the brain are among the advantages provided by compositions of the present invention.

## Claims

1. A composition which comprises a liposome coupled to an antibody binding fragment which binds to a receptor molecule present on vascular endothelial cells of a mammalian blood-brain barrier.

2. The composition of claim 1, wherein the antibody binding fragment is Fab, F(ab')₂, Fab' or a single antibody chain polypeptide.

3. The composition according to claim 1, wherein the antibody fragment binds to a receptor of the brain peptide transport system.

4. The composition according to claim 3, wherein the receptor is transferrin receptor, insulin receptor, IGF-I or IGF-2 receptor.

5. The composition according to claim 1, wherein the antibody binding fragment is coupled by a covalent bond to the liposome.

6. The composition according to claim 1, wherein the liposome contains a pharmaceutical compound.

7. The composition of claim 6, wherein the pharmacologic compound is a hydrophilic neuropeptide.

8. The composition according to claim 6, further comprising a physiologically acceptable carrier.

9. The composition according to claim 1, wherein the diameter of the liposome is less than 1 micron.

10. The composition according to claim 9, wherein the diameter of the liposome is smaller than about 0.45 microns.

11. The composition of claim 1, wherein the antibody binding fragment is Fab, F(ab')₂ or Fab' which binds specifically to transferrin receptor.

12. The composition of claim 1, wherein the antibody binding fragment is prepared from a monoclonal antibody.

13. A pharmaceutical composition which comprises an antibody binding fragment which binds to a receptor molecule present on vascular endothelial cells of a mammalian blind-brain barrier, wherein the antibody binding fragment is coupled to a liposome which contains a pharmaceutical compound intended for treatment of a brain-associated disorder, and a pharmaceutically acceptable carrier.

14. The pharmaceutical composition of claim 13, wherein the antibody binding fragment is Fab, F(ab')₂ Fab' or a single antibody chain polypeptide.

15. The pharmaceutical composition of clam 14, wherein the receptor is transferrin receptor, insulin receptor, IGF-I or IGF-2 receptor.

16. Use of a composition which comprises a liposome containing a pharmacological compound coupled to an antibody binding fragment which binds to a receptor molecule present on vascular endothelial cells of a mammalian blood-brain barrier, for the manufacture of a medicament for targeting said pharmacological compound for delivery to the blood-brain barrier of said mammal.

17. Use of a composition as claimed in claim 16, wherein the composition is to be administered intravenously or intraarterially.

18. Use of a composition as claimed in claim 16 wherein the antibody binding fragment binds specifically to transferrin receptor, insulin receptor, IGF-I or IGF-2 receptor.

19. Use or a composition as claimed in claim 16, wherein the antibody binding fragment is Fab, F(ab')₂, Fab' or a single antibody chain polypeptide.

20. Use of a composition as claimed in claim 16, wherein the receptor is the transferrin receptor.

## Patentansprüche

1. Zusammensetzung, die ein mit einem Antikörper-Bindungsfragment gekoppeltes Liposom enthält, das an ein an vaskulären Endothelzellen der Blut-Hirn-Schranke eines Säugetiers vorhandenes Rezeptor-Molekül bindet.

2. Zusammensetzung nach Anspruch 1, wobei das Antikörper-Bindungsfragment Fab, F(ab')₂, Fab' oder eine einzelne Antikörper-Polypeptidkette ist.

3. Zusammensetzung nach Anspruch 1, wobei das Antikörper-Fragment an einen Rezeptor des Hirn-Peptid-Transportsystems bindet.

4. Zusammensetzung nach Anspruch 3, wobei der Rezeptor der Transferrin-Rezeptor, Insulin-Rezeptor, IGF-I- oder IGF-II-Rezeptor ist.

5. Zusammensetzung nach Anspruch 1, wobei das Antikörper-Bindungsfragment durch eine unpolare Bindung mit dem Liposom gekoppelt ist.

6. Zusammensetzung nach Anspruch 1, wobei das Liposom eine pharmazeutische Verbindung enthält.

7. Zusammensetzung nach Anspruch 6, wobei die pharmakologische Verbindung ein hydrophiles Neuropeptid ist.

8. Zusammensetzung nach Anspruch 6, die weiterhin eine physiologisch verträgliche Trägersubstanz enthält.

9. Zusammensetzung nach Anspruch 1, wobei der Durchmesser des Liposoms geringer als 1 Mikrometer ist.

10. Zusammensetzung nach Anspruch 9, wobei der Durchmesser des Liposoms kleiner als etwa 0,45 Mikrometer ist.

11. Zusammensetzung nach Anspruch 1, wobei das spezifisch an den Transferrin-Rezeptor bindende Antikörper-Bindungsfragment Fab, F(ab')₂ oder Fab' ist.

12. Zusammensetzung nach Anspruch 1, wobei das Antikörper-Bindungsfragment aus einem monoklonalen Antikörper hergestellt ist.

13. Pharmazeutische Zusammensetzung, die ein Antikörper-Bindungsfragment enthält, das an ein an vaskulären Endothelzellen einer Blut-Hirn-Schranke eines Säugetiers vorhandenes Rezeptor-Molekül bindet, wobei das Antikörper-Bindungsfragment mit einem Liposom gekoppelt ist, das eine zur Behandlung einer das Hirn betreffenden Erkrankung vorgesehene pharmazeutische Verbindung und eine pharmazeutisch verträgliche Trägersubstanz enthält.

14. Pharmazeutische Zusammensetzung nach Anspruch 13, wobei das Antikörper-Bindungsfragment Fab, F(ab')₂, Fab' oder eine einzelne Antikörper-Polypeptidkette ist.

15. Pharmazeutische Zusammensetzung nach Anspruch 144 wobei der Rezeptor der Transferrin-Rezeptor, Insulin-Rezeptor, IGF-I- oder IGF-II-Rezeptor ist.

16. Verwendung einer ein Liposom enthaltenden Zusammensetzung zur Herstellung eines Medikaments zur zielgerichteten Zuführung der pharmakologischen Verbindung zur Blut-Hirn-Schranke des Säugetiers, wobei das Liposom eine mit einem Antikörper-Bindungsfragment gekoppelte pharmakologische Verbindung enthält, wobei das Antikörper-Bindungsfragment an ein an vaskulären Endothelzellen einer Blut-Hirn-Schranke eines Säugetiers vorhandenes Rezeptor-Molekül bindet.

17. Verwendung einer Zusammensetzung nach Anspruch 16, wobei die Zusammensetzung intravenös oder intraarteriell verabreicht werden soll.

18. Verwendung einer Zusammensetzung nach Anspruch 16, wobei das Antikörper-Bindungsfragment an den Transferrin-Rezeptor, Insulin-Rezeptor, IGF-I- oder IGF-II-Rezeptor spezifisch bindet.

19. Verwendung einer Zusammensetzung nach Anspruch 16, wobei das Antikörper-Bindungsfragment Fab, F(ab')₂, Fab' oder eine einzelne Antikörper-Polypeptidkette ist.

20. Verwendung einer Zusammensetzung nach Anspruch 16, wobei der Rezeptor der Transferrin-Rezeptor ist.

## Revendications

1. Composition qui comprend un liposome couplé à un fragment liant d'anticorps qui se lie à une molécule de récepteur présente sur les cellules endothéliales vasculaires de la barrière hémato-encéphalique d'un mammifère.

2. Composition de la Revendication 1, dans laquelle le fragment liant d'anticorps est un fragment Fab, F(ab')₂, Fab' ou un polypeptide de chaîne d'anticorps unique.

3. Composition selon la Revendication 1, dans laquelle le fragment d'anticorps se lie à un récepteur du système de transport des peptides cérébraux.

4. Composition selon la Revendication 3, dans laquelle le récepteur est le récepteur de la transferrine, le récepteur de l'insuline, le récepteur de l'IGF-1 ou de l'IGF-2.

5. Composition selon la Revendication 1, dans laquelle le fragment liant d'anticorps est couplé au liposome par une liaison covalente.

6. Composition selon la Revendication 1, dans laquelle le liposome contient un composé pharmaceutique.

7. Composition de la Revendication 6, dans laquelle le composé pharmacologique est un neuropeptide hydrophile.

8. Composition selon la Revendication 6, comprenant en outre un support physiologiquement acceptable.

9. Composition selon la Revendication 1, dans laquelle le diamètre du liposome est inférieur à 1 micron.

10. Composition selon la Revendication 9, dans laquelle le diamètre du liposome est inférieur à environ 0,45 micron.

11. Composition de la Revendication 1, dans laquelle le fragment liant d'anticorps est un fragment Fab, F(ab')₂ ou Fab' qui se lie spécifiquement au récepteur de la transferrine.

12. Composition de la Revendication 1, dans laquelle le fragment liant d'anticorps est préparé à partir d'un anticorps monoclonal.

13. Composition pharmaceutique qui comprend un fragment liant d'anticorps qui se lie à une molécule de récepteur présente sur les cellules endothéliales vasculaires de la barrière hémato-encéphalique d'un mammifère, dans laquelle le fragment liant d'anticorps est couplé à un liposome qui contient un composé pharmaceutique destiné au traitement d'un trouble en rapport avec le cerveau, et un support pharmaceutiquement acceptable.

14. Composition pharmaceutique de la Revendication 13, dans laquelle le fragment liant d'anticorps est un fragment Fab, F(ab')₂, Fab' ou un polypeptide de chaîne d'anticorps unique.

15. Composition pharmaceutique de la Revendication 14, dans laquelle le récepteur est le récepteur de la transferrine, le récepteur de l'insuline, le récepteur de l'IGF-I ou de l'IGF-2.

16. Utilisation d'une composition qui comprend un liposome contenant un composé pharmacologique couplé à un fragment liant d'anticorps qui se lie à une molécule de récepteur présente sur les cellules endothéliales vasculaires de la barrière hémato-encéphalique d'un mammifère, pour la fabrication d'un médicament permettant le ciblage dudit composé pharmacologique pour sa délivrance à la barrière hémato-encéphalique dudit mammifère.

17. Utilisation d'une composition selon la Revendication 16, dans laquelle la composition est à administrer par voie intraveineuse ou intra-artérielle.

18. Utilisation d'une composition selon la Revendication 16, dans laquelle le fragment liant d'anticorps se lie spécifiquement au récepteur de la transterrine, au récepteur de l'insuline, au récepteur de l'IGF-I ou de l'IGF-2.

19. Utilisation d'une composition selon la Revendication 16, dans laquelle le fragment liant d'anticorps est un fragment Fab, F(ab')₂, Fab' ou un polypeptide de chaîne d'anticorps unique.

20. Utilisation d'une composition selon la Revendication 16, dans laquelle le récepteur est le récepteur de la transferrine.
